Europäisches Patentamt

European Patent Office

Office européen des brevets

(11) Veröffentlichungsnummer: **0 013 334**
**A2**

(12) **EUROPÄISCHE PATENTANMELDUNG**

(21) Anmeldenummer: **79104858.0**

(22) Anmeldetag: **04.12.79**

(51) Int. Cl.³: **A 61 M 5/14**

(30) Priorität: **22.12.78 DE 2855713**

(43) Veröffentlichungstag der Anmeldung:
**23.07.80 Patentblatt 80/15**

(84) Benannte Vertragsstaaten:
**CH FR GB SE**

(71) Anmelder: **Doehn, Manfred, Dr.**
**Schmuckshöhe 9**
**D-2000 Hamburg 63(DE)**

(72) Erfinder: **Doehn, Manfred, Dr.**
**Schmuckshöhe 9**
**D-2000 Hamburg 63(DE)**

(74) Vertreter: **Hansmann, Dierk**
**Jessenstrasse 4**
**D-2000 Hamburg 50(DE)**

(54) Vorrichtung zur Infusion von Lösungen aus mehreren Infusionsflaschen.

(57) Zur Zuführung von Infusionslösungen aus mehreren Infusionsflaschen (1,2,3,4) ist in jeder Zuflußleitung (9, 10, 11, 12) ein Absperrventil (5,6,7,8) angeordnet, wobei alle Zuflußleitungen (9,10,11,12) in einer Sammelleitung (14) zusammengefaßt sind. Diese Sammelleitung (14) ist mit einer Pumpe (15) zur Zuführung zum Patienten verbunden. Über einen Rechner (19) werden Öffnungszeiten der einzelnen Absperrventile (9,10,11,12) ermittelt und entsprechend nacheinander über ein Steuergerät (20) geschaltet, so daß durch die Öffnungszeiten die Fördermenge bestimmt wird und aus jeder Infusionsflasche (1,2,3,4) nacheinander eine schubweise Infusion der einzelnen Infusionslösungen erfolgt.

FIG.1

EP 0 013 334 A2

0013334

## Vorrichtung zur Infusion von Lösungen aus mehreren Infusionflaschen

Die Erfindung bezieht sich auf eine Vorrichtung zur Infusion von Lösungen aus mehreren Infusionsflaschen, wobei der Zufluß aus den Infusionsflaschen über ein Stellglied beeinflußbar ist.

Bei der Behandlung eines Patienten besteht das Problem, daß mehrere, z.B. drei bis vier Infusionen verschiedener Lösungen in unterschiedlichen Mengen gleichzeitig über einen venösen Zugang zugeführt werden müssen. Die gewünschte Einlaufmenge pro Stunde wird hierbei je Infusionsflasche über Rollklemmen entsprechend individuell von Hand eingestellt. Die derart eingestellten Mengen werden etwa stündlich abgelesen und müssen häufig kontrolliert werden, wobei gegebenenfalls eine Korrektur der Einstellung

0013334

vorgenommen werden muß. Dieses Verfahren nimmt in der Praxis viel Zeit des Pflegepersonals in Anspruch und gewährt keinen Schutz dagegen, daß eine Lösung einmal zu schnell oder zum anderen zu langsam zugeführt wird.

Weiterhin muß berücksichtigt werden, daß bei unterschiedlich gefüllten bzw. bei unterschiedlich hoch hängenden Infusionsflaschen immer andere Zulaufverhältnisse vorliegen, die sich während der Infusion noch ändern.

Es ist bekannt, Infusionen mit Hilfe von speziellen peristaltischen Pumpen dem Patienten zuzuführen. Von Nachteil ist bei dieser Methode, daß für eine einzelne Infusionslösung eine eigene derartige Pumpe eingesetzt werden muß. Die Bereithaltung der notwendigen Anzahl von Pumpen ist jedoch sehr kostspielig.

Die Aufgabe der Erfindung ist es, einfache Anordnungen zu schaffen, die es jeweils ermöglichen, mehrere Infusionslösungen in medizinisch einwandfreier Weise zu infundieren und dabei insbesondere eine stets definierte Zuführung zu gewährleisten.

Die Lösung dieser Aufgabe erfolgt erfindungsgemäß jeweils durch die Maßnahmen und Merkmale in den kennzeichnenden Teilen der Ansprüche 1 und 2.

Weitere Ausführungen der Erfindung sind in den Unteransprüchen festgelegt.

Hierdurch wird der Vorteil geschaffen, daß einmal durch die Pumpe eine Fördergeschwindigkeit unabhängig von der Füllung und beispielsweise der Viskosität der Flüssigkeit/gewährleistet ist. Weiterhin wird

0013334

zyklisch innerhalb einer bestimmten Zeitdauer, z.B. in vier Minuten, aus jeder Infusionsflasche anteilmäßig die verordnete Menge infundiert. Die kontinuierliche Infusion mehrerer Lösungen zugleich wird hierbei durch eine kurzzeitig nacheinander erfolgende, schubweise Infusion einzelner Flüssigkeiten mit geringen Mengen ersetzt. Durch die zyklische Folge der einzelnen Öffnungszeiten der Absperrventile erfolgt somit eine Aufteilung, die hohe Konzentrationen bestimmter Substanzen vermeidet.

Die gemäß dem Anspruch 1 charakterisierte Infusionsvorrichtung führt die Infusion in kurzperiodischer Weise mittels pro Periode mehrerer, verschieden lang andauernder Entnahmen der einzelnen Lösungen bei gleichförmiger Pumpgeschwindigkeit durch. Im Unterschied dazu läuft die Infusion mittels der gemäß Anspruch 2 beschriebenen Infusionsvorrichtung als ebenfalls kurzperiodische Infusion ab, jedoch mit gegebenenfalls unterschiedlichen Einzelöffnungszeiten der Absperrventile und darauf entsprechend abgestimmten Einzelentnahmen mit gegebenenfalls unterschiedlichen Entnahmegeschwindigkeiten der Pumpe.

Die Einstellung des Steuergerätes zur Betätigung der einzelnen Absperrventile und zur Einhaltung der Pumpenförderleistung oder -leistungen unter Berücksichtigung der Zeit T für einen Zyklus wird gemäß der Erfindung durch den Rechner in hohem Maße vereinfacht. Es brauchen lediglich die gewünschten Werte eingestellt zu werden, woraufhin die Infusion zuverlässig abläuft. Herkömmliche Wächtereinrichtungen sorgen für Schutz vor Störungsfolgen.

Eine Zykluszeit von wenigen Minuten, z.B. 4 Minuten ist medizinisch unbedenklich. Selbstverständlich sind auch Signaleinrichtungen für die Anzeige von Störungen und die Einleitung von Gegenmaßnahmen vorgesehen.

Die Erfindung wird im folgenden anhand von Beispielen mit Hilfe der Figuren in der Zeichnung näher erläutert. Es zeigen:

Fig. 1 das Blockschema der erfindungsgemäßen Infusionsvorrichtung;

Fig. 2 ein Blockschema einer Infusionssteuerung gemäß Anspruch 1;

Fig. 3 ein Blockschema einer Infusionssteuerung gemäß Anspruch 2.

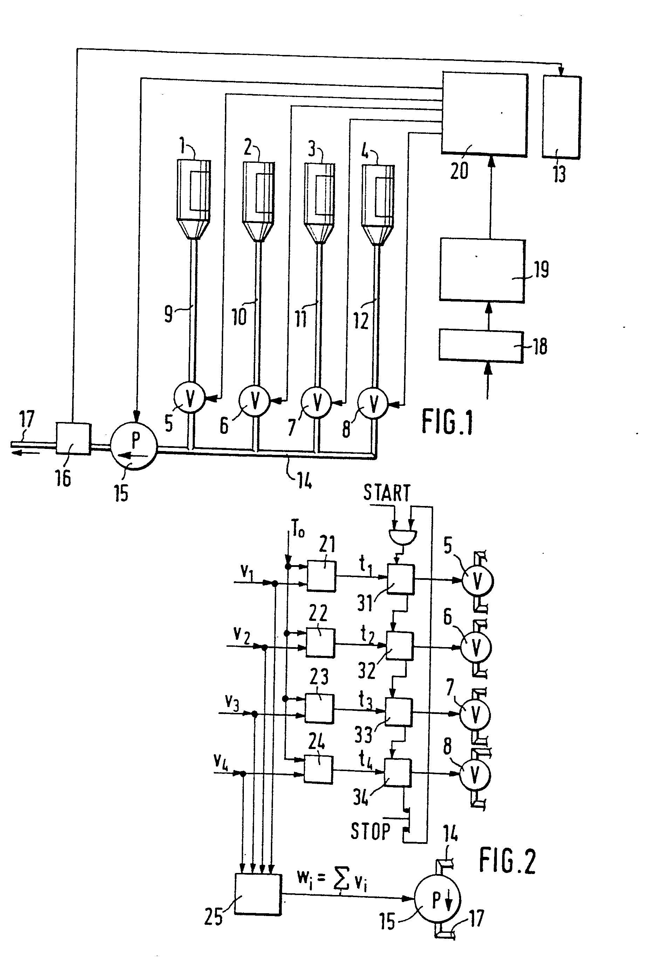

In Fig. 1 sind 4 Flaschen 1,2,3,4 mit den verschiedenen Infusionslösungen dargestellt. In Absperrventile 5,6,7 und 8 in Form von Abklemmvorrichtungen sind die Schläuche der Zuflußleitungen 9,10, 11 und 12 eingelegt, die hinter den Ventilen 5,6,7 und 8 in eine Sammelleitung 14 zusammengeführt sind. Diese ist üblicherweise als Schlauch ausgebildet und durch die besondere Pumpe 15 geführt, welche vorzugsweise eine peristaltische Pumpe ist. An der Ausgangsseite der Pumpe 15 befindet sich eine Wächtereinrichtung 16, mittels der Störungen, z.B. Luftblasen, über eine Anzeige 13 signalisiert werden können. Der Ausgang 17 der Sammelleitung 14 führt zum Patienten. Die vom Arzt vorgeschriebenen Werte für die Zyklusdauer T der Infusion und für die Langzeit-Infusionsrate V (i = 1,2...n) der n einzelnen Infusionslösungen (in diesem Beispiel n = 4) werden in die Eingabeinheit 18 eingegeben, ferner noch die Anzahl n der Lösungen und gegebenenfalls sogenannte Steuervariable $a_i$, deren Sinn später erläutert wird.

Ein Rechner 19 nimmt die Werte auf und erzeugt für die Einzelfälle i = 1,2,...n Ausgangswerte $t_i$ für die Einzelöffnungszeiten der Ventile 5,6,7 und 8 und $w_i$ für die momentanen Förderleistungen der Pumpe 15, die zu den jeweiligen $t_i$ gehören. Diese Ausgangswerte werden dem Steuergerät 20 übermittelt, welches die Ventile 5,6,7 und 8 in zyklischer Folge innerhalb der Zeit $T_o$ einzeln öffnet, während die Pumpe 15 jeweils die Förderleistung $w_i$ aufbringt, die ebenfalls von dem Steuergerät 20 abgegeben wird.

In dem Fall, daß die Pumpe 15 stets die gleiche Förderleistung aufbringen soll und entsprechend die Einzel-öffnungszeiten $t_i$ der Ventile 5,6,7 und 8 nach Maßgabe der vorgeschriebenen Langzeit-Infusionsraten $V_i$ verschieden lang andauern, berechnen sich die $t_i$ nach der Formel

$$t_i = T_o \cdot V_i : (V_1 + V_2 + ... + V_n)$$ und die konstante Förderleistung $W_i = W_1 = W_2 = ... = W_n = V_1 + V_2 + ... V_n$.

Diese Infusionsweise ist in der Fig. 2 dargestellt, in der die Blockschema-Elemente 21,22,23 und 24 Multiplizierglieder 31,32,33 und 34 in zyklischer Serie geschaltete Zeitgeberelemente sind und 25 ein Summierglied ist, während die sonstigen Elemente und Größen die vorstehend erläuterte Bedeutung haben.

Nach der Erfindung ist auch eine Infusionsweise möglich und vorgesehen, bei der die Einzelöffnungszeiten $t_i$ der Ventile 5,6,7 und 8 untereinander gleich und dafür zur Einhaltung der vorgeschriebenen Langzeit-Infusions-raten $V_i$ die jeweiligen Förderleistungen $w_i$ der Pumpe 15 unterschiedlich sind. Ferner ist es innerhalb dieses Infusionsablaufes noch möglich, wahlweise die Werte $w_i$ einzeln zu ändern, wobei die zugehörigen Einzelöff-nungszeiten $t_i$ unter Beibehaltung der Zyklusdauer $T_o$ selbsttätig angepaßt werden.

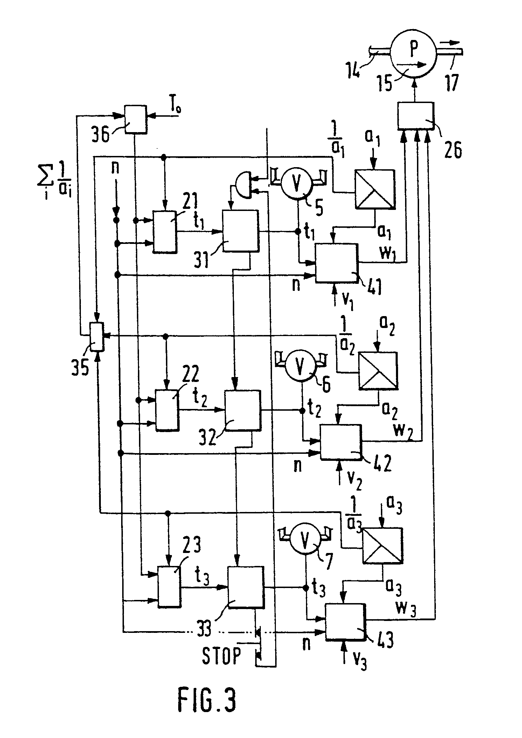

In Fig. 3 ist in gleicher Bezeichnungsweise der
Blockschemaglieder wie in Fig. 2 das entsprechende
Blockschema der Infusionsvorrichtung für ein
Beispiel mit 3 Infusionslösungen dargestellt. Über
jeweils ein Variierglied 41,42 bzw. 43 kann zum
Zwecke der Variation der momentanen Förderleistungen
$w_i$ ein Faktor $a_i$ einmultipliziert werden, welcher
bewirkt, daß sich die momentane Förderleistung
$w_i$ der Pumpe 15 in bestimmtem Maße einstellbar
ändert. Im reziproken Maße hierzu muß die zugehörige Einzelöffnungszeit $t_i$ geändert werden, welches
durch Multiplikation der Zeit $t_i$ mit dem Faktor $1/a_i$
erreicht wird. Der jeweilige Zeitgeber 31, 32 bzw. 33
erhält also den neuen Wert für $t_i$. Da sich durch diese
Maßnahme zunächst auch die Zykluszeit $T_o$ ändert, muß
allen Zeitgebern 31, 32 und 33 der Faktor
$(1/a_1 + 1/a_2 + \ldots + 1/a_n)$, der im Funktionsglied 35 gebildet wird, eingegeben werden. Er
normiert die durch Variation der $w_i$ geänderte Zykluszeit wieder auf den ursprünglichen Wert $T_o$. Im
Produktglied 36 wird dieser Normierfaktor der Zeit
$T_o$ hinzugefügt. Im Leistungsverstärker 26 werden die
eigentlichen Regelwerte für die momentanen Förderleistungen der Pumpe 15 gebildet. Im Blockschema sind
die entsprechenden Signalwege und Signalgrößen direkt
angegeben.

Ein konkretes Zahlenbeispiel kann dann bei einem 4-Minuten-Zyklus wie folgt aussehen:

Infusionsflasche 1  60 ml/h (Glukose)     = $V_1$
           "      2  20 ml/h (Aminosäure) = $V_2$
           "      3  10 ml/h (Albumin)   = $V_3$
           "      4  10 ml/h (KCl)       = $V_4$

dem entspricht:

Gesamtmenge / Zeit       $V_i$ = 100 ml/h
Einzelmenge/ Zeit        $V_i$ =  60, 20, 10, 10 ml/h
Öffnungszeit eines Zyklus $T_0$ = 240 sec
Öffnungszeit für Einzelmenge 1   144 sec = $t_1$
                                   2    48 sec = $t_2$
                                   3    24 sec = $t_3$
                                   4    24 sec = $t_4$

Im Rahmen der Erfindung liegt es auch, andere als die vorstehend beschriebenen Infusionssteuerungen vorzusehen, z.B. solche mit Lochstreifeneinrichtungen.

Patentansprüche

1. Vorrichtung zur Infusion von Lösungen aus mehreren Infusionsflaschen, wobei der Zufluß aus den Infusionsflaschen über ein Stellglied beeinflußbar ist, dadurch gekennzeichnet, daß jede Zuflußleitung (9,10,11,12) einer Infusionsflasche (1,2,3,4) ein Absperrventil (5,6,7,8) aufweist und alle Zuflußleitungen (9,10,11,12) in eine Sammelleitung (14) zusammengeführt sind, in die eine Pumpe (15) eingeschaltet ist, und daß ein Rechner (19) die in ihn über Eingabeelemente (18) eingebbaren, wählbaren Werte für eine Zykluszeit $T_o$ und für die Langzeit-Infusionsraten $V_i$ (i = 1,2...n, n = Anzahl der Lösungen) der n einzelnen Lösungen zu Steuerwerten für die Förderleistung der Pumpe (15) und für die einzelnen Öffnungszeiten $t_i$ (i = 1,2...n) der Absperrventile (5,6,7,8) verarbeitet, welche Steuerwerte von einem Steuergerät (20) zur Zeit- und Folgesteuerung der Pumpe (15) und der Absperrventile (5,6,7,8) aufnehmbar sind, wobei das Steuergerät (20) die Pumpe (15) zu einer Förderleistung gleich der Summe der Infusionsraten $V_i$ anregt und von den Absperrventilen (5,6,7,8) nacheinander und zyklisch jeweils eines (z.B. 5) während der zugehörigen Öffnungszeit $t_i = T \cdot V_i : (V_1 + V_2 + \ldots + V_n)$, $i = 1,2,\ldots n$ öffnet, während es die übrigen Absperrventile (in diesem Beispiel dann 6,7 und 8) geschlossen hält.

2. Vorrichtung zur Infusion von Lösungen aus mehreren Infusionsflaschen, wobei der Zufluß aus den Infusionsflaschen über ein Stellglied beeinflußbar ist, dadurch gekennzeichnet, daß jede Zuflußleitung (9,10,11,12) einer Infusionsflasche (1,2,3,4) ein Absperrventil (5,6,7,8) aufweist und alle Zuflußleitungen (9,10,11,12) in eine Sammelleitung (14) zusammengeführt sind, in die eine Pumpe (15) eingeschaltet ist, und daß ein Rechner (19) die in ihn über Eingabeelemente (18) eingebbaren, wählbaren Werte für die Einzelöffnungszeiten $t_i$ ($i = 1,2,\ldots n$) der Absperrventile (5,6,7,8), für eine Zykluszeit $T_o$ und für die Langzeit-Infusionsraten $V_i$ ($i = 1,2\ldots n$) zu Steuerwerten für die einzelnen Öffnungszeiten $t_i$, wobei $T_o = (t_1 + t_2 + \ldots + t_n)$ ist, und für die zugehörigen Förderleistungen $W_i$ ($i = 1,2,\ldots n$) der Pumpe (15) verarbeitet, die von einem Steuergerät (20) zur Zeit- und Folgesteuerung der Pumpe (15) und der Absperrventile (5,6,7,8) aufnehmbar sind, wobei das Steuergerät (20) von den Absperrventilen (5,6,7,8) nacheinander und zyklisch jeweils eines (z.B. 5) während der zugehörigen Öffnungszeit $t_i$ öffnet und dabei die übrigen Absperrventile (in diesem Beispiel dann 6,7 und 8) geschlossen hält und zugleich die Pumpe (15) zum Fördern mit der zugehörigen Förderleistung $W_i$ ($i = 1,2,\ldots n$) anregt.

3. Vorrichtung nach Anspruch 2, dadurch gekennzeichnet, daß für die Einzelöffnungszeiten $t_i$ in allen Fällen $i = 1,2,...n$ der gleiche Wert wählbar ist.

4. Vorrichtung nach einem der Ansprüche 1 bis 3, dadurch gekennzeichnet, daß der Rechner (19) ein programmierbarer, elektronischer Rechner und das Steuergerät (20) eine in elektronischer und elektrischer Schaltweise aufgebaute Einheit ist.

5. Vorrichtung nach einem der Ansprüche 1 bis 4, dadurch gekennzeichnet, daß die Pumpe (15) eine Schlauchpumpe ist.

0013334

FIG.1

FIG.2

$w_i = \sum v_i$

FIG.3